# FASCICULE DE BREVET EUROPEEN

(11) **EP 1 026 170 B1**
(45) Date de publication et mention de la délivrance du brevet: **15.01.2003**
(21) Numéro de dépôt: 00400286.1
(22) Date de dépôt: 03.02.2000
(51) Int. Cl.: C07H 17/08, A61K 31/70

(54) **Nouveaux dérivés de l'erythromycine, leur procédé de préparation et leur application comme médicaments**
Erythromycin-Derivate, ihr Verfahren zur Herstellung und ihre Verwendung als Arzneimittel
Erythromycin derivatives, their preparation and use as medicaments

(30) Priorité: 04.02.1999 FR 9901292
(43) Date de publication de la demande: 09.08.2000
(73) Titulaire: Aventis Pharma S.A., 92160 Antony (FR)
(72) Inventeur: Denis, Alexis, 75011 Paris (FR); Fromentin, Claude, 75019 Paris (FR); Heckmann, Bertrand, 94230 Cachan (FR)

(56) Documents cités:
- EP-A- 0 676 409
- EP-A- 0 680 967
- EP-A- 0 799 833
- FR-A- 2 732 023
- FR-A- 2 732 684

## Description

La présente invention concerne de nouveaux dérivés de l'érythromycine, leur procédé de préparation et leur application comme médicaments.

Les demandes de brevet EP-A-0680967 et FR-A-2732684 décrivent des dérivés 11, 12- carbamates cycliques de la 11-déoxy-3- de[(2,6-didéoxy-3-C-méthyl-3-0-méthyl-α-L-ribohexopyranosyl)oxy]-6-0-méthyl-3-oxoérythromycine dont l'atome d'azote du carbamate est substitué par une chaîne (CH₂)ₙ-Ar, n'étant égal à 3, 4 ou 5 et Ar pouvant être un hétéroaryle choisi parmi une liste limitative d'hétérocycles.

Les demandes de brevets EP-A-0676409 et FR-A-2732023 décrivent des dérivés du même type, dont l'azote du carbamate est substitué par un groupe NR₁R₂, dans lequel R₁ et R₂ peuvent notamment former avec l'atome d'azote un hétérocycle à caractère aromatique lequel peut être substitué.

Enfin, la demande EP-A-0799833 décrit des dérivés du même type, dont l'atome d'azote du carbamate est substitué par une chaîne X-Y-Ar, X représentant notamment CH₂, Y représentant -(CH₂)ₘ-(CH=CH)ₙ-(CH₂)ₒ-, m+n+o étant inférieur ou égal à 8 et n'étant O ou 1, et Ar pouvant être un hétérocycle à caractère aromatique, lequel peut être substitué.

L'invention a pour objet, les composés de formule (I) ainsi que leurs sels d'addition avec les acides : 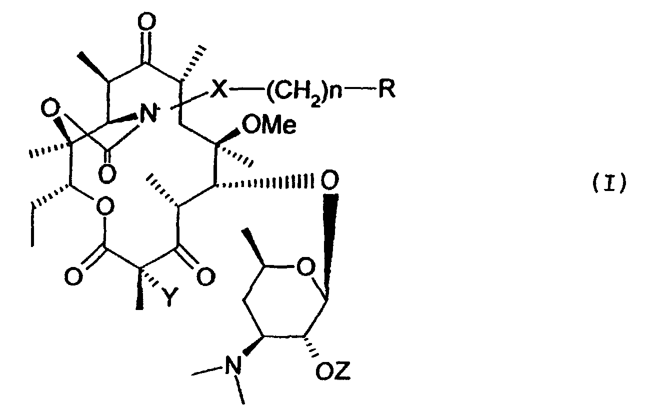 dans lesquels X représente un radical CH₂ ou NH, n représente un nombre entier compris entre 1 et 8,
Y représente un atome d'hydrogène ou d'halogène, R représente un radical : 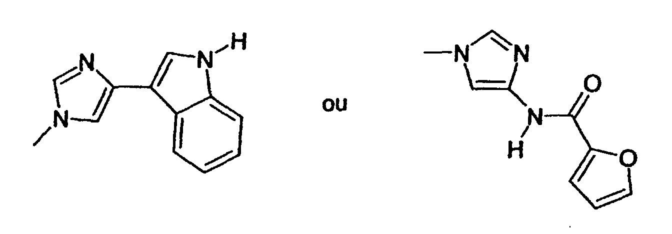 et Z représente un atome d'hydrogène ou le reste d'un acide.

Parmi les sels d'addition avec les acides, on peut citer les sels formés avec les acides acétique, propionique, trifluoroacétique, maléique, tartrique, méthanesulfonique, benzènesulfonique, p-toluènesulfonique et spécialement les acides stéariques, éthylsuccinique ou laurylsulfonique.

Lorsque Y représente un atome d'halogène, il s'agit par exemple, d'un atome de fluor, de chlore ou de brome.

L'invention a plus particulièrement pour objet les composés de formule (I) dans lesquels X représente un radical CH₂, ceux dans lesquels Y représente un atome d'hydrogène, ceux dans lesquels Y représente un atome de fluor, ceux dans lesquels n représente le nombre 4.

L'invention a plus particulièrement pour objet les composés dont la préparation est donnée ci-après dans la partie expérimentale, et tout particulièrement les produits des exemples 1 et 3.

Les produits de formule générale (I) possèdent une très bonne activité antibiotique sur les bactéries gram^{⊕} telles que les staphylocoques, les streptocoques, les pneumocoques.

Les composés de l'invention peuvent donc être utilisés comme médicaments dans le traitement des infections à germes sensibles et notamment, dans celui des staphylococcies malignes de la face ou cutanées, pyodermites, plaies septiques ou suppurantes, furoncles, anthrax, phlegmons, érysipèles et acné, staphylococcies telles que les angines aiguës primitives ou post-grippales, bronchopneumonies, suppuration pulmonaires, les streptococcies telles que les angines aiguës, les otites, les sinusites, la scarlatine, les pneumococcies telles que les pneumonies, les bronchites ; la brucellose, la diphtérie, la gonococcie.

Les produits de la présente invention sont également actifs contre les infections dues à des germes comme Haemophilus influenzae, Rickettsies, Mycoplasma pneumoniae, Chlamydia, Legionella, Ureaplasma, Toxoplasma ou à des germes du genre Mycobactérium.

La présente invention a donc également pour objet, à titre de médicaments et, notamment de médicaments antibiotiques, les produits de formule (I) tels que définis ci-dessus, ainsi que leurs sels d'addition avec les acides minéraux ou organiques pharmaceutiquement acceptables.

L'invention a plus particulièrement pour objet, à titre de médicaments et, notamment de médicaments antibiotiques, les produits des exemples 1 et 3 et leurs sels pharmaceutiquement acceptables.

L'invention a également pour objet les compositions pharmaceutiques renfermant comme principe actif au moins un des médicaments définis ci-dessus.

Ces compositions peuvent être administrées par voie buccale, rectale, parentérale ou par voie locale en application topique sur la peau et les muqueuses, mais la voie d'administration préférée est la voie buccale.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 300 mg par jour par voie orale, chez l'adulte pour le produit de l'exemple 1.

L'invention a également pour objet un procédé de préparation caractérisé en ce que l'on soumet un composé de formule (II) : 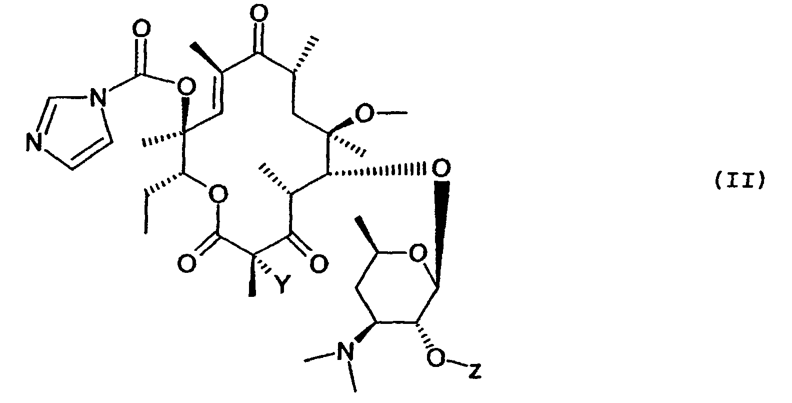 dans laquelle Y conserve sa signification précédente et Z a la définition précitée, à l'exception d'hydrogène, à l'action d'un composé de formule (III) :

R(CH₂)ₙXNH₂ (III)

dans lequel R, n et X conservent la même signification que précédemment pour obtenir le composé de formule (I) correspondant, dans lequel Z représente le reste d'un acide, puis éventuellement à l'action d'un agent de déprotection de la fonction hydroxyle en 2' pour obtenir le composé de formule (I) dans lequel Z représente un atome d'hydrogène et/ou à l'action d'un acide pour en former le sel.

Les composés de formule (II) dans lesquels Y représente un atome d'hydrogène, utilisés comme produits de départ sont décrits et revendiqués dans le brevet Européen EP 0596802.

Les produits de formule (II) dans lesquels Y est un halogène sont décrits et revendiqués dans la demande de brevet européen 0949268. Il est décrit ci-après un exemple détaillé de préparation de composés de formule (I) dans lequel Hal représente un atome de fluor. Ce procédé peut être schématisé comme suit : on soumet le composé A, 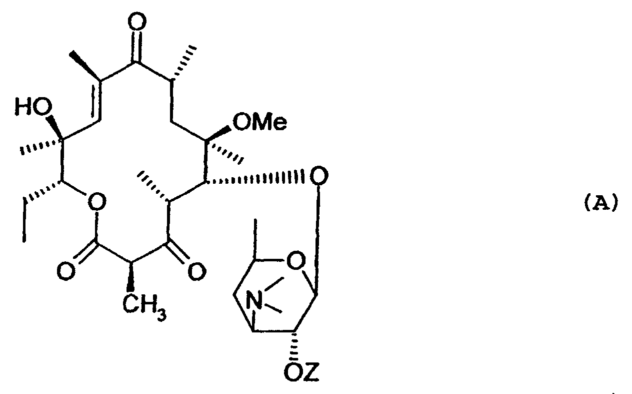 dans laquelle OZ représente un radical OH libre ou protégé, à l'action d'un agent de fluoration pour obtenir le composé de formule (B) correspondant : 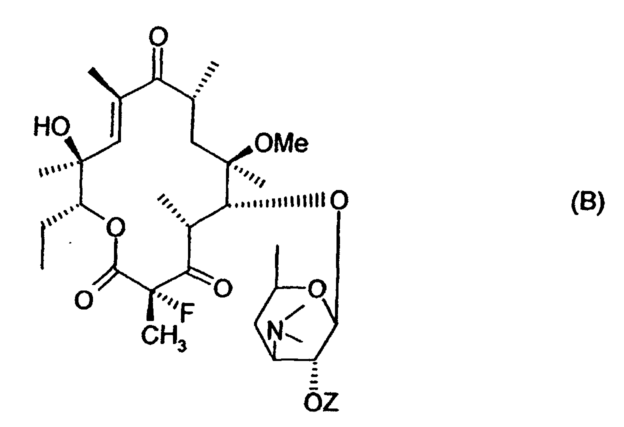 que l'on soumet à l'action du carbonyldiimidazole, pour obtenir le composé de formule (II) correspondant dans lequel Hal représente un atome de fluor.
Les composés de formule (III) utilisés comme produits de départ sont nouveaux et sont en eux-mêmes un objet de la présente invention. Leur préparation est donnée ci-après dans la partie expérimentale.

L'invention a donc également pour objet à titre de produits chimiques nouveaux, les produits de formule (III) tels que définis précédemment et notamment les produits de formule (III) dans lesquels n représente le nombre 3 et X représente un radical CH₂.

Les exemples suivants illustrent l'invention sans toutefois la limiter.

### EXEMPLE 1 : 11,12-didéoxy-3-de [(2,6-didéoxy-3-C-méthyl-3-O-méthyl-.alpha.-L-ribo-hexopyranosyl)oxy]-6-O-méthyl-3-oxo-12,11-[oxycarbonyl[[4-[4-(1H-indol-3-yl)-1H-imidazol-1-yl]butyl]-imino]]érythromycine.

On agite à 70°C pendant 48 heures un mélange de 3,78 g de l'amine obtenue à la préparation **1**, 25 ml d'acétonitrile, 2,5 ml d'eau et 4,2 g de 2'-acétoxy de 12-(oxycarbonyl-imidazol)-11-déoxy-10,11-didéhydro-3-de [2,6-didéoxy-3-C-méthyl-3-O-méthyl-α-L-ribohexopyranosyl)oxy]6-O-méthyl-3-oxo-érythromycine. On évapore à sec. On ajoute 30 ml de méthanol et agite 3 heures au reflux. On évapore à sec. On chromatographie le produit obtenu sur silice en éluant avec le mélange acétate d'éthyle-éthanol-triéthylamine 90-5-5. On obtient 2,2 g de produit que l'on purifie sur silice en éluant avec le mélange chloroforme-méthanol-ammoniaque 96-4-0,5. On obtient 1,73 g d'un produit que l'on reprend dans l'acétate d'éthyle, lave avec une solution aqueuse d'ammoniaque à 1 %, sèche, filtre et évapore à sec. On empâte dans l'éther éthylique, essore et sèche. On obtient 1,440 g de produit recherché.
**Spectre RMN (400 MHz dans CDCl**_{**3**}**)**
H2 : 3.86 ppm ; H4 ; 3.08 ppm ; H5 : 4,25 ppm ; H7 : 1.60 1.81 ppm ; H8 : 2.60 ppm ; H10 : 3.13 ppm ; H11 : 3.58 ppm ; H13 : 4.95 ppm ; H14 : 1.56 1.96 ; H15 : 0.83 ppm ; 2Me : 1.38 ppm ; 4Me : 1.31 ppm ; 6Me : 1.34 ou 1.47 ppm ; 8Me : 1.15 ppm ; 10Me : 1.00 ppm ; 12Me : 1.34 ou 1.47 ppm ; 6OMe : 2.65 ppm ; 1' : 4.29 ppm ; 2' : 3.19 ppm ; 3' : 2.45 ppm ; 4' : 1.66 et 1.23 ppm ; 5' : 3.53 ppm ; NMe₂ : 2.26 ppm ; NCH₂ : 3.65 3.76 ppm ; CH₂ : 1.68 1.91 ppm ; CH₂N : 3.99 ppm ; imidazole : 7.23 7.53 ppm ; indole : 8.60 7.67 7.39 7.18 7.90 ppm.
**Spectre de masse**
MH⁺ : 850⁺
Désosamine : 158⁺
M-H⁻ : 848⁻

### EXEMPLE 2 : 11,12-didéoxy-3-de [(2,6-didéoxy-3-C-méthyl-3-O-méthyl-.alpha.-L-ribo-hexopyranosyl)oxy]-2-fluoro-6-O-méthyl-3-oxo-12,11-[oxycarbonyl[[4-[4-(1H-indol-3-yl)-1H-imidazol-1-yl]-butyl]-imino]]-érythromycine.

En opérant comme à l'exemple 1, à partir du composé de formule (II) 2-fluoré obtenu comme indiqué à la préparation 2 ci-après, on a obtenu le produit recherché.
**Spectre RMN (400 MHz dans CDCl**_{**3**}**)**
H4 : 3.53 ppm ; H5 : 4.07 ppm ; H7 : 1.52 1.85 ppm ; H8 : 2.62 ppm ; H10 : 3.19 ppm ; H11 : 3.44 ppm ; H13 : 4.89 ppm ; H14 : 1.63 1.98 ; 15Me : 0.87 ppm ; 2Me : 1.79 ppm 4Me : 1.32 ppm ; 6Me : 1.34 ou 1.50 ppm ; 8Me : 1.16 ppm ; 10Me : 1.01 ppm ; 12Me : 1.34 ou 1.50 ppm ; 6OMe : 2.57 ppm ; 1' : 4.31 ppm ; 2' : 3.20 ppm ; 3' : 2.48 ppm ; 4' : 1.68 et 1.24 ppm ; 5' : 3.53 ppm ; NMe₂ : 2.28 ppm ; CH₂ : 1.66 1.86 ppm ; CH₂N : 3.74 3.69 ppm.
**Spectre de masse**
MH⁺ : 868⁺

### EXEMPLE 3 : 11,12-didéoxy-3 de [(2,6-didéoxy-3-C-méthyl-3-O-méthyl-.alpha.-L-ribo-hexopyranosyl)-oxy]-6-O-méthyl-3-oxo-12,11-[oxycarbonyl-[[4-[4-[(2-furanylcarbonyl)-amino]-1H-imidazol-1-yl]-butyl]-imino]]-érythromycine.

En opérant comme précédemment on a obtenu le produit recherché.
rf = 0,25 (CH₂Cl₂ CH₃OH NH₄OH 95-5-04)
**Spectre RMN CDCl**_{**3**}
H2 : 3,85 ppm ; H4 : 3,07 ppm ; H5 4,24 ppm ; H7 : 1,58 et 1,84 ppm ; H8 : 2,62 ppm ; H10 : 3,12 ppm ; H11 : 3,56 ppm ; H13 : 4,91 ppm ; H14 : 1,60-1,97 ppm ; H15 : 0,85 ppm ; 2Me : 1,25 ppm ; 4Me : 1,30 ppm ; 6Me : 1,36 ou 1,47 ppm ; 8Me : 1,17 ppm ; 10Me : 1,00 ppm ; 12Me : 1,36 ou 1,47 ppm ; 6 OMe: 2,63 ppm ; 1' : 4,28 ppm ; 2' : 3,20 ppm ; 3' : 2,48 ppm ; 4' : 1,67 et 1,23 ppm ; 5' : 3,56 ppm ; 5Me : 1,25 ppm ; N(Me)₂ : 2,28 ppm ; NCH₂ : 3,96 ppm ; CH₂ : 1,84 ppm ; CH₂ : 1,62 ppm ; CH₂N : 3,63 et 3,32 ppm ; H2, H5 : 7,39 et 7,28 ppm ; H3 : 7,18 ppm ; H4 : 6,53 ppm ; H5 : 7,50 ppm ; NHC=O : 8,30 ppm.

### EXEMPLE 4 : 11,12-didéoxy-3-de [(2,6-didéoxy-3-C-méthyl-3-O-méthyl-.alpha.-L-ribo-hexopyranosyl)oxy]-2-fluoro-6-O-méthyl-3-oxo-12,11-[oxycarbonyl-[[4-[4-(2-furanylcarbonyl)-amino]-1H-imidazol-1-yl]-butyl]-imino]]-érythromycine.

En opérant comme précédemment, on a obtenu le produit recherché.
**Spectre RMN CDCl**_{**3**}
H4 : 3,54 ppm ; H5 4,07 ppm ; H7 : 1,51 et 1,87 ppm ; H8 : 2,62 ppm ; H10 : 3,10 ppm ; H11 : 3,42 ppm ; H13 : 4,86 ppm ; H14 : 1,65-1,98 ppm ; H15 : 0,89 ppm ; 2Me : 1,78 (d) ppm ; 4Me : 1,31 ppm ; 6Me : 1,36 ou 1,50 ppm ; 8Me : 1,19 ppm ; 10Me : 1,01 ppm ; 12Me : 1,36 ou 1,50 ppm ; 6 Ome : 2,55 ppm ; 1' : 4,31 ppm ; 2' : 3,20 ppm ; 3' : 2,48 ppm ; 4' : 1,69 et 1,24 ppm ; 5' : 3,54 ppm ; 5'Me : 1,24 ppm ; N(Me)₂ : 2,29 ppm ; NCH₂ : 3,58-3,71 ppm ; CH₂ : 1,62 ppm ; CH₂ : 1,83 ppm ; CH₂N : 3,94 ppm; imidazole : 7,27-7,39 ppm ; NH : 8,65 ppm ; furanne H3 : 7,18 ppm ; H4 : 6,52 ppm ; H5 : 7,49 ppm.

### préparation 1 : 4-(1H-indol-3-yl)-1H-imidazole-1-butanamine

### Stade A : 3-(1H-imidazole-4-yl)-1H-indole

On agite à 180°C pendant une heure 12,87 g de 2-bromo-1-(1H-indol-3-yl)-éthanone et 27,4 ml de formamide. On reprend dans l'eau, lave à l'acétate d'éthyle, amène à pH supérieur à 10 avec une solution d'ammoniaque à 5 %, extrait à l'acétate d'éthyle, sèche et évapore à sec. On chromatographie sur silice en éluant avec le mélange chlorure de méthylène-méthanol-ammoniaque 96-4-0,5. On obtient 2,53 g de produit recherché.

### Stade B : 2-[4-[4-(1H-indol-3-yl)1H-imidazol-1-yl]butyl]-1H-isoindole-1,3-(2H)-dione.

On agite à 110°C pendant 15 minutes 7,9 g de produit du stade A, 50 ml de DMF et 11,93 g de carbonate de potassium, sèche à l'étuve sous anhydride phosphorique. On ajoute goutte à goutte à température ambiante une solution de 15,83 g de N-(4-bromobutyl)phtalimide dans 30 ml de DMF. On agite une heure à 110°C. On verse sur du phosphate acide de sodium. On évapore sous pression réduite. On obtient 19,11 g de produit brut que l'on purifie sur silice en éluant avec le mélange chlorure de méthylène-méthanol-ammoniaque 97-3-05. On obtient 4,58 g de produit recherché.

### Stade C : 4-(1H-indol-3-yl)-1H-imidazole-1-butanamine

On agite pendant 24 heures au reflux un mélange de 4,58 g de produit du stade précédent, 200 ml d'éthanol et 1,45 ml d'hydrate d'hydrazine. On concentre à 30 ml, filtre, rince à l'éthanol et évapore à sec. On obtient 3,78 g de produit recherché.

### Préparation 2 : 2'-acétoxy-2α-fluoro de 12-(oxycarbonylimidazol)-11-déoxy-10,11-didéhydro-3-de[2,6-didéoxy-3-C-méthyl-3-O-méthyl-α-L-ribohexopyranosyl)-oxy]-6-O-méthyl-3-oxo-érythromycine.

### Stade A : 11-déoxy-10,11-didéhydro-3-de[(2,6-didéoxy-3-C-méthyl-3-0-méthyl-α-L-ribohexopyranosyl)oxy]-6-O-méthyl-3-oxo-érythromycine.

On agite pendant 44 heures un mélange de 8,722 g de 2'-acétoxy de 11-déoxy-10,11-didéhydro-3-de[(2,6-didéoxy-3-C-méthyl-3-O-méthyl-α-L-ribohexopyranosyl)oxy]-6-O-méthyl-3-oxo-érythromycine (EP 596802) et 350 ml de méthanol anhydre. On évapore, reprend au chlorure de méthylène, sèche et obtient 8,794 g du produit recherché.

### Stade B : 2'-triméthylsilyloxy de 11-déoxy-10,11-didéhydro-3-de[(2,6-didéoxy-3-méthyl-3-O-méthyl-α-L-ribohexopyranosyl)-oxy]-6-O-méthyl-3-oxo-érythromycine.

On agite à température ambiante pendant 4 jours un mélange renfermant 3,08 g du produit du stade précédent, 340 mg d'imidazole, 32 ml de THF anhydre et 1,06 ml d'hexaméthyldisilazane. On évapore à sec, reprend avec un mélange de 60 ml de chlorure de méthylène et de 60 ml d'une solution aqueuse de phosphate acide de sodium 0,5 M. On maintient le mélange sous agitation pendant 15 minutes, décante, extrait au chlorure de méthylène, sèche et évapore et sec. On obtient 3,345 g du produit recherché.

### Stade C : 2'-triméthylsilyloxy-2α-fluoro de 11-déoxy-10,11-didéhydro-3-de[(2,6-didéoxy-3-C-méthyl-3-O-méthyl-α-L-ribohexopyranosyl)-oxy]-6-O-méthyl-3-oxo-érythromycine.

On ajoute à -12°C sous atmosphère d'argon 1,24 ml d'une solution de terbutylate de potassium dans le THF 0,97 M dans une solution renfermant 668 mg de 2'-triméthylsilyloxy de 11-déoxy-10,11-didéhydro-3-de[(2,6-didéoxy-3-O-méthyl-α-L-ribohexopyranosyl)oxy]-6-O-méthyl-3-oxo-érythromycine et 6,7 ml de THF anhydre. On agite 5 minutes et ajoute 378 mg de N-fluorodibenzènesulfonimide. On agite 10 minutes à -12°C et laisse revenir à la température ambiante pendant 1 heure 30 minutes. On obtient 695 mg du produit recherché.

### Stade D : 2α-fluoro de 11-déoxy-10,11-didéhydro-3-de[(2,6-didéoxy-3-C-méthyl-3-O-méthyl-α-L-ribohexopyranosyl)oxy]-6-O-méthyl-3-oxo-érythromycine.

On agite pendant 3 heures 30 minutes un mélangé de 5,476 g de produit du stade précédent, 50 ml de THF et 11,2 ml de fluorure de tétrabutylammonium 1M dans le THF. On évapore le solvant et ajoute 37 ml d'acétate d'éthyle, 37 ml d'eau et 7,5 ml d'ammoniaque à 20 %. On agite 10 minutes, décante, extrait à l'acétate d'éthyle, sèche, filtre et concentre à sec le filtrat. On chromatographie le produit obtenu sur silice en éluant avec le mélange CH₂Cl₂-MeOH ammoniaqué 99-1, puis 98-2, 97-3, 96-4, 95-5. On obtient 2,452 g de produit recherché.

### Stade E : 2'-acétoxy-2α-fluoro de 11-déoxy-10,11-didéhydro-3-de[(2,6-didéoxy-3-O-méthyl-α-L-ribohexopyranosyl)oxy]-6-O-méthyl-3-oxo-érythromycine.

On maintient sous agitation pendant 3 heures 1,02 g de produit du stade A, 10 ml de chlorure de méthylène et 241 µl d'anhydride acétique. On évapore et ajoute 10 ml d'eau et 10 ml d'acétate d'éthyle. On laisse 1 heure à la température ambiante sous agitation, décante, sèche et évapore. On obtient 1,01 g de produit recherché.

### Stade F : 2'-acétoxy-2α-fluoro de 12-(oxycarbonylimidazol)-11-déoxy-10,11-didéhydro-3-de[(2,6-didéoxy-3-C-méthyl-3-O-méthyl-α-L-ribohexopyranosyl)oxy]-6-O-méthyl-3-oxoérythromycine.

On ajoute à 0°C, 0,388 g de carbonyldiimidazole et 24 µl de DBU dans une solution renfermant 1,01 g du produit du stade précédent et 10 ml de THF anhydre. On maintient le mélange réactionnel sous agitation à 0°C pendant 19 heures. On évapore le THF et ajoute 10 ml d'eau et 10 ml d'acétate d'éthyle. On maintient le mélange réactionnel sous agitation pendant 10 minutes, extrait, sèche et évapore. On obtient 0,902 g de produit recherché brut que l'on chromatographie en éluant avec un mélange acétate d'éthyle-triéthylamine 96-4. On obtient 0,573 g de produit recherché.

### Préparation 3 : N- [1-(4-aminobutyl)-1H-imidazol-4-yl]-2-furancarboxamide

### Stade A : 2-[4- (4-nitro-1H-imidazol-1-yl)butyl]-1H-isoindole-1,3(2H)dione.

On porte au reflux pendant 2 heures un mélange de 15 g de 4-nitro-1H-imidazole, 250 ml de DMF, 26,95 g de carbonate de potassium, et 37,41 g de 2-(4-bromobutyl)-1H-isoindole-1,3(2H)dione. On laisse revenir à la température ambiante et verse sur l'eau. On filtre, rince et sèche. On obtient 33,31 g de produit recherché.

### Stade B : 4-nitro-1H-imidazole-1-butanamine.

On porte au reflux pendant 20 heures un mélange de 33,31 g de produit du stade A, 555 ml d'éthanol et 11,33 ml d'hydrazine. On filtre, laisse revenir à la température ambiante et amène à sec. On obtient 33,04 g de produit.

### Stade C : [4-(4-nitro-1H-imidazol-1-yl)butyl]-carbamate de 1,1-diméthyléthyle.

On agite pendant 1,30 heures à la température ambiante 33,04 g de produit du stade précédent, 665,8 ml de THF, 222,32 ml d'eau, 19,2 ml de triéthylamine, et 27,76 g d'anhydride terbutylique. On concentre sous pression réduite et reprend à l'eau. On essore, lave et sèche sous pression réduite à 60°C. On obtient 20,3 g de produit recherché.

### Stade D : [4-(4-amino-1H-imidazol-1-yl)butyl]-carbamate de 1,1-dimétyléthyle.

On hydrogène un mélange de 19,25 g de produit du stade précédent, 5,77 ml d'éthanol et 19 g de nickel de Raney. On filtre et rince à l'éthanol sous atmosphère d'azote. On obtient 21,38 g de produit recherché.

### Stade E : [4-[4-[(2-furanylcarbonyl)amino-1H-imidazol-1-yl)butyl]-carbamate de 1,1-dimétyléthyle.

On agite pendant 5 minutes sous atmosphère d'azote un mélange de 21,38 g de produit du stade précédent, 962 ml de THF et 14,19 ml de triéthylamine. On amène à 0°C et introduit 7,52 ml du chlorure de furan-2-oyle carbonyle. On laisse revenir à la température ambiante, agite pendant 2 heures. On verse sur l'eau, agite et ajoute 500 ml d'acétate d'éthyle. On agite, décante et extrait à l'acétate d'éthyle. On lave la phase organique à l'eau, sèche, filtre et amène à sec. On obtient 25,18 g de produit recherché.

### Stade F : N-[1-(4-aminobutyl)-1H-imidazol-4-yl]-2-furancarboxamide.

On amène à 0°C une solution de 25,18 g de produit du stade précédent et 126 ml de chlorure de méthylène. On ajoute goutte à goutte 103 ml de TFA. On laisse revenir à la température ambiante et agite pendant 1 heure 30. On évapore le chlorure de méthylène et reprend à l'éther. On agite 1 heure en présence d'éther. On filtre et sèche. On obtient un produit que l'on purifie sur résine. On obtient 9,4 g de produit recherché.

### EXEMPLE DE COMPOSITION PHARMACEUTIQUE

On a préparé des comprimés renfermant :
Produit de l'exemple 1 150 mg
Excipient q.s.p. 1 g

Détail de l'excipient : amidon, talc, stéarate de magnésium

### ETUDE PHARMACOLOGIQUE DES PRODUITS DE L'INVENTION

### Méthode des dilutions en milieu liquide.

On prépare une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de vingt-quatre heures à l'étuve à 37°C, l'inhibition de la croissance est. appréciée par transillumination, ce qui permet de déterminer les concentrations minimales inhibitrices (C.M.I) exprimés en microgrammes/cm³.

Les résultats suivants ont été obtenus (lecture après 24 heures).

| **PRODUITS DE L'EXEMPLE :** | | | | |
|---|---|---|---|---|
| N° | | | 1 | 3 |
| 01 | S. aureus | 011UC4 | 0.040 | 0.150 |
| 02 | S. aureus | 011UC4+ sérum 50% | 0.040 | 0.150 |
| 03 | S. aureus | 011B18c | | |
| 04 | S. aureus | 011GR12c | | |
| 05 | S. aureus | 011GO25i | 0.080 | 0.150 |
| 06 | S. epidermidis | 012GO11i | 0.080 | 0.40 |
| 07 | S. aureus | 011CB20c | | |
| 08 | S. epidermidis | 012GO40c | | |
| 09 | S. pyogènes | 02A1UC1 | 0.02 | 0.150 |
| 10 | S. agalactiae | 02B1HT1 | 0.02 | 0.02 |
| 11 | S. faecalis | 02D2UC1 | 0.040 | 0.040 |
| 12 | S. faecium | 02D3HT1 | 0.02 | 0.040 |
| 13 | Streptococcus gr. G | 02GOGR5 | 0.040 | 0.040 |
| 14 | S. mitis | 02MitCB1 | 0.02 | 0.040 |
| 15 | S. pyogènes | 02A1SJ1c | 5.000 | 10.000 |
| 16 | S. agalactiae | 02B1SJ1c | 0.080 | 0.040 |
| 17 | S. faecalis | 02D2DU15c | 10.000 | 2.500 |
| 18 | Streptococcus gr. G | 02GOgr4c | 20.000 | 10.000 |
| 19 | S. sanguis | 02SgGr10i | 0.150 | 0.040 |
| 20 | S. mitis | 02MitGR16i | 0.02 | 0.02 |
| 21 | S. pneumoniae | 032UC1 | 0.02 | 0.080 |
| 22 | S. pneumoniae | 030GR20 | 0.02 | 0.02 |
| 23 | S. pneumoniae | 030SJ5i | 0.040 | 0.02 |
| 24 | S. pneumoniae | 030CR18c | 0.600 | 0.300 |
| 25 | S. pneumoniae | 030PW23C | 0.080 | 0.040 |
| 26 | S. pneumoniae | 030RO1i | 0.080 | 0.080 |
| 27 | S. pneumoniae | 030SJ1C | 0.080 | 0.080 |

## Revendications

1. Les composés de formule (I) ainsi que leurs sels d'addition avec les acides : 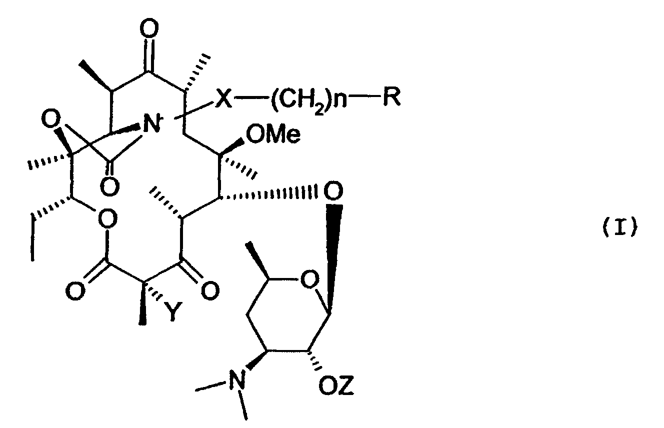 dans lesquels X représente un radical CH₂ ou NH, n représente un nombre entier compris entre 1 et 8,
Y représente un atome d'hydrogène ou d'halogène, R représente un radical : 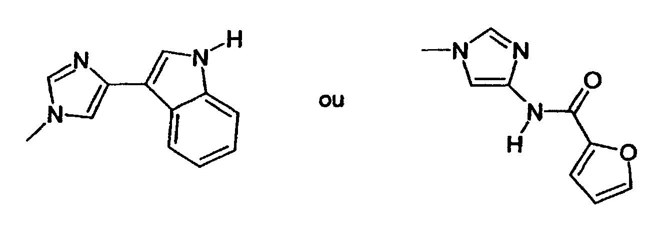 et Z représente un atome d'hydrogène ou le reste d'un acide.

2. Les composés de formule (I) définis à la revendication 1, dans lesquels X représente un radical CH₂.

3. Les composés de formule (I) définis à la revendication 1 ou 2 dans lesquels Y représente un atome d'hydrogène.

4. Les composés de formule (I) définis à la revendication 1 ou 2, dans lesquels Y représente un atome de fluor.

5. Les composés de formule (I) définis à l'une quelconque des revendications 1 à 4, dans lesquels n représente le nombre 4.

6. Les composés de formule (I) définis à la revendication 1
- 11,12-didéoxy-3-de [(2,6-didéoxy-3-C-méthyl-3-O-méthyl-.alpha.-L-ribo-hexopyranosyl)oxy]-6-O-méthyl-3-oxo-12,11-[oxycarbonyl[[4-[4-(1H-indol-3-yl)-1H-imidazol-1-yl]-butyl]-imino]]-érythromycine,
- 11,12-didéoxy-3-de [(2,6-didéoxy-3-C-méthyl-3-O-méthyl-.alpha.-L-ribo-hexopyranosyl)oxy]-6-O-méthyl-3-oxo-12,11-[oxycarbonyl[[4-[[4-(2-furanylcarbonyl)amino]-1H-imidazol-1-yl]-butyl]-imino]]-érythromycine.

7. A titre de médicaments les composés de formule (I) définis à l'une quelconque des revendications 1 à 6, ainsi que leurs sels pharmaceutiquement acceptables.

8. Les compositions pharmaceutiques renfermant comme principe actif au moins un médicament défini à la revendication 7.

9. Procédé de préparation des compositions de formule (I) défini à l'une quelconque des revendications 1 à 6, **caractérisé en ce que** l'on soumet un composé de formule (II) : 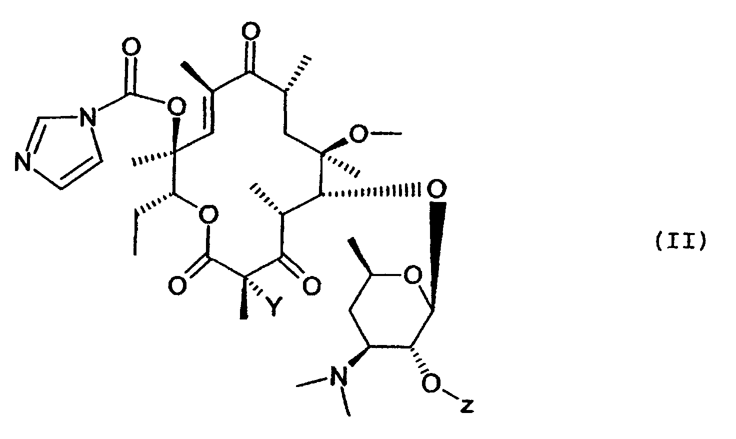 dans laquelle Y conserve la même signification que dans la revendication 1 et Z à la même signification que dans la revendication 1, à l'exception d'hydrogène, à l'action d'un composé de formule (III) :
R(CH₂)ₙXNH₂ (III)
dans lequel R, n et X conservent la même signification que dans la revendication 1, pour obtenir le composé de formule (I) correspondant, dans lequel Z représente le reste d'un acide, puis éventuellement à l'action d'un agent de déprotection de la fonction hydroxyle en 2' pour obtenir le composé de formule (I) dans lequel Z représente un atome d'hydrogène et/ou à l'action d'un acide pour en former le sel.

10. A titre de produits chimiques nouveaux les composés de formule (III) définis à la revendication 9.

11. Les composés de formule (III) définis à la revendication 10 dans lesquels n représente le nombre 3 et X un radical CH₂.

## Patentansprüche

1. Verbindungen der Formel (I) sowie ihre Additionssalze mit Säuren: 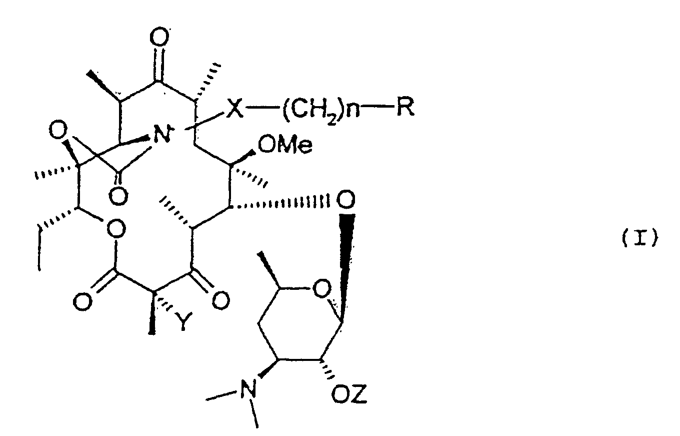 in der X einen Rest CH₂ oder NH darstellt, n eine ganze Zahl zwischen 1 und 8 ist,
Y ein Wasserstoffatom oder ein Halogenatom bedeutet, R ein Rest 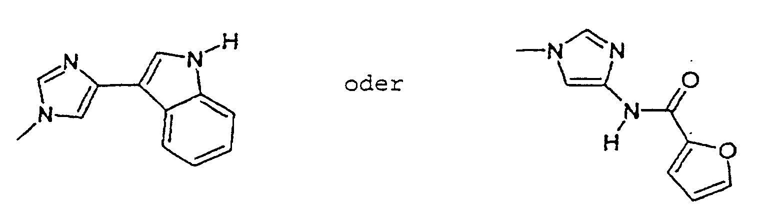 ist und Z ein Wasserstoffatom oder den Rest einer Säure darstellt.

2. Verbindungen der Formel (I) wie in Anspruch 1 definiert, worin X ein Rest CH₂ ist.

3. Verbindungen der Formel (I) wie in Anspruch 1 oder 2 definiert, worin Y ein Wasserstoffatom ist.

4. Verbindungen der Formel (I) wie in Anspruch 1 oder 2 definiert, worin Y ein Fluoratom ist.

5. Verbindungen der Formel (I) wie in irgendeinem der Ansprüche 1 bis 4 definiert, worin n die Zahl 4 darstellt.

6. Verbindungen der Formel (I) wie in Anspruch 1 definiert
- 11,12-Dideoxy-3-de-[(2,6-dideoxy-3-C-methyl-3-O-methyl-.alpha.-L-ribohexopyranosyl)-oxy]-6-O-methyl-3-oxo-12,11-{oxycarbonyl[[4-(4-(1H-indol-3-yl)-1H-imidazol-1-yl)-butyl]-imino]}-erythromycin,
- 11,12-Dideoxy-3-de-[(2,6-dideoxy-3-C-methyl-3-O-methyl-.alpha.-L-ribohexopyranosyl)-oxy]-6-O-methyl-3-oxo-12,11-{oxycarbonyl{[4-[(4-(2-furanylcarbonyl)-amino)-1H-imidazol-1-yl]-butyl]-imino}}-erythromycin.

7. Als Arzneimittel die Verbindungen der Formel (I) wie in irgendeinem der Ansprüche 1 bis 6 definiert, sowie ihre pharmazeutisch akzeptablen Salze.

8. Pharmazeutische Zusammensetzungen, umfassend als Wirkstoff mindestens ein Arzneimittel wie in Anspruch 7 definiert.

9. Verfahren zur Herstellung der Verbindungen der Formel (I) wie in irgendeinem der Ansprüche 1 bis 6 definiert, **dadurch gekennzeichnet, daß** man eine Verbindung der Formel (II) 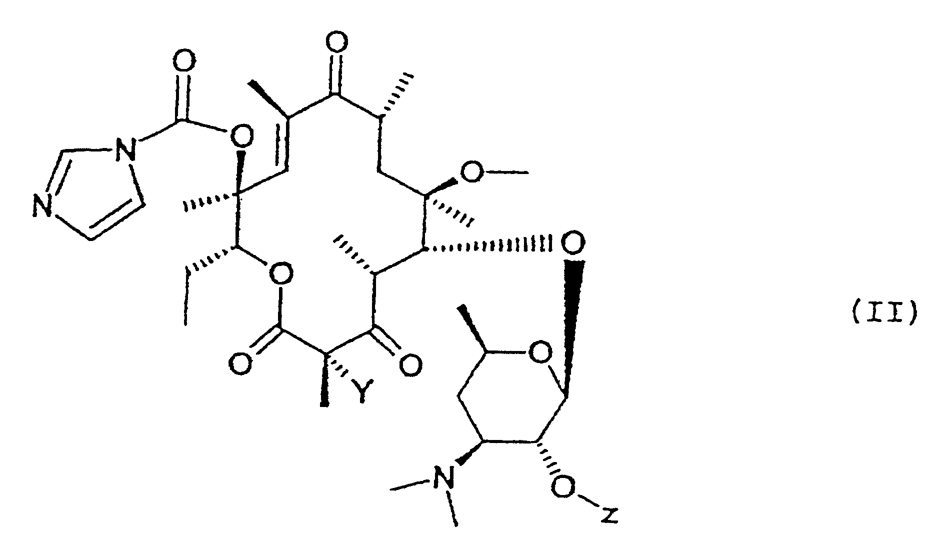 in der Y die gleiche Bedeutung wie in Anspruch 1 beibehält und Z die gleiche Bedeutung wie in Anspruch 1 hat, mit Ausnahme von Wasserstoff, der Einwirkung einer Verbindung der Formel (III)
R(CH₂)ₙXNH₂ (III)
unterzieht, in der R, n und X die gleiche Bedeutung wie in Anspruch 1 beibehalten, um die entsprechende Verbindung der Formel (I) zu erhalten, in der Z den Rest einer Säure darstellt, und anschließend gegebenenfalls der Einwirkung eines Mittels zum Abspalten der Schutzgruppe von der Hydroxylfunktion in 2' unterzieht, um die Verbindung der Formel (I) zu erhalten, in der Z ein Wasserstoffatom ist und/oder der Einwirkung einer Säure unterwirft, um das Salz zu bilden.

10. Als neue chemische Produkte die Verbindungen der Formel (III) wie in Anspruch 9 definiert.

11. Verbindungen der Formel (III) wie in Anspruch 10 definiert, worin n die Zahl 3 ist und X einen Rest CH₂ darstellt.

## Claims

1. The compounds of formula (I) as well as their addition salts with acids: 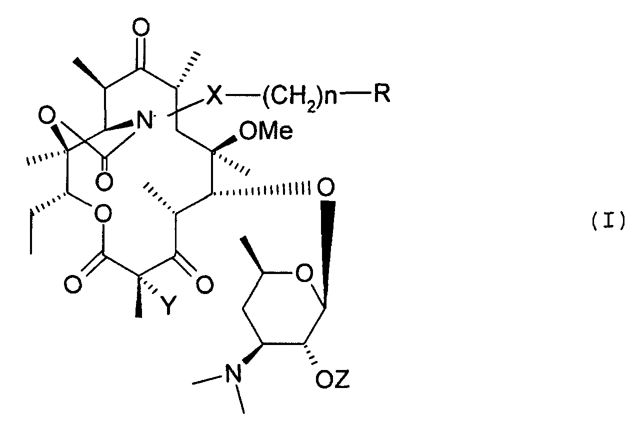 in which X represents a CH₂ or NH radical, n represents an integer comprised between 1 and 8,
Y represents a hydrogen or halogen atom, R represents a: 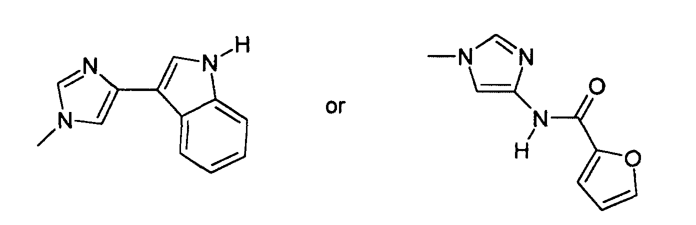 radical and Z represents a hydrogen atom or the remainder of an acid.

2. The compounds of formula (I) defined in claim 1, in which X represents a CH₂ radical.

3. The compounds of formula (I) defined in claim 1 or 2 in which Y represents a hydrogen atom.

4. The compounds of formula (I) defined in claim 1 or 2, in which Y represents a fluorine atom.

5. The compounds of formula (I) defined in any one of claims 1 to 4, in which n represents the number 4.

6. The compounds of formula (I) defined in claim 1
- 11,12-dideoxy-3-de[(2,6-dideoxy-3-C-methyl-3-O-methyl-.alpha.-L-ribo-hexopyranosyl)oxy]-6-O-methyl-3-oxo-12,11-[oxycarbonyl[[4-[4-(1H-indol-3-yl)-1H-imidazol-1-yl]-butyl]-imino]]-erythromycin,
- 11,12-dideoxy-3-de[(2,6-dideoxy-3-C-methyl-3-O-methyl-.alpha.-L-ribo-hexopyranosyl)oxy]-6-O-methyl-3-oxo-12,11-[oxycarbonyl[[4-[(4-(2-furanylcarbonyl)amino]-1H-imidazol-1-yl]-butyl]-imino]]-erythromycin.

7. As medicaments, the compounds of formula (I) defined in any one of claims 1 to 6, as well as their pharmaceutically acceptable salts.

8. The pharmaceutical compositions containing, as active ingredient, at least one medicament defined in claim 7.

9. Process for the preparation of the compositions of formula (I) defined in any one of claims 1 to 6, **characterized in that** a compound of formula (II): 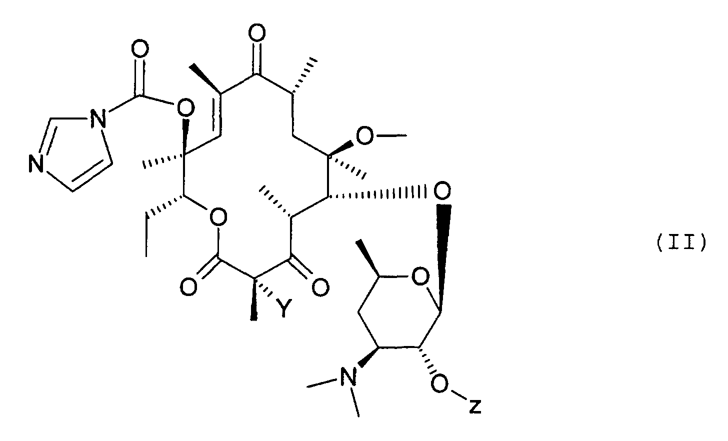 in which Y retains the same meaning as in claim 1 and Z has the same meaning as in claim 1, except for hydrogen, is subjected to the action of a compound of formula (III):
R(CH₂)ₙXNH₂ (III)
in which R, n and X retain the same meaning as in claim 1, in order to obtain the corresponding compound of formula (I), in which Z represents the remainder of an acid, then optionally to the action of a deprotection agent of the hydroxyl function in position 2' in order to obtain the compound of formula (I) in which Z represents a hydrogen atom and/or to the action of an acid in order to form the salt.

10. As new chemical products, the compounds of formula (III) defined in claim 9.

11. The compounds of formula (III) defined in claim 10 in which n represents the number 3 and X represents a CH₂ radical.
